## Europäisches Patentamt
## European Patent Office
## Office européen des brevets

(19)

(11) Veröffentlichungsnummer : **0 131 950**
**B1**

(12)

# EUROPÄISCHE PATENTSCHRIFT

(45) Veröffentlichungstag der Patentschrift :
23.11.89

(21) Anmeldenummer : 84108355.3

(22) Anmeldetag : 16.07.84

(51) Int. Cl.⁴ : **A 61 K   9/20**

(54) **Schnell resorbierbare Zubereitung von Glibenclamid.**

(30) Priorität : 19.07.83 DE 3325986

(43) Veröffentlichungstag der Anmeldung :
23.01.85 Patentblatt 85/04

(45) Bekanntmachung des Hinweises auf die Patenterteilung : 23.11.89 Patentblatt 89/47

(84) Benannte Vertragsstaaten :
AT BE CH DE FR GB IT LI LU NL SE

(56) Entgegenhaltungen :
EP--A-- 0 086 468
DE--A-- 2 355 743
DE--A-- 3 151 196

(73) Patentinhaber : Klinge Pharma GmbH
Berg-am-Laim-Strasse 129
D-8000 München 80 (DE)

(72) Erfinder : Hofer, Josef Maximilian
Wasserburgerstrasse 24a
D-8011 Kirchseeon (DE)
Erfinder : Stanislaus, Fritz
Halserspitzstrasse 12
D-8000 München 80 (DE)

(74) Vertreter : Wuesthoff, Franz, Dr.-Ing. et al
Patentanwälte Wuesthoff -v. Pechmann-Behrens-
Goetz Schweigerstrasse 2
D-8000 München 90 (DE)

**Beschreibung**

Es ist seit langem bekannt, daß die Resorbierbarkeit von oral zu applizierenden schwer löslichen Arzneimittelwirkstoffen von der Teilchengröße abhängt, in der der Wirkstoff nach Zerfall der Tablette oder des Dragees bzw. der Kapsel vorliegt. Je feiner die Teilchengröße, desto leichter kann im Magen- und Darmtrakt nach der Einnahme eine beschleunigte Auflösung und Resorption des Wirkstoffs erreicht werden. In der DE-OS 20 17 495 ist beschrieben, daß durch Einbetten von schwer löslichen Sulfonamiden mit Teilchengröße unter 5 μm in Galaktomannanen eine orale Applikationsform erhalten wird, mit der eine schnellere Resorption des Sulfonamids erreichbar ist. Auch ist es schon seit längerem bekannt, daß Netzmittel die Löslichkeit und damit auch die Resorbierbarkeit derartiger Wirkstoffe verbessern. Dementsprechend wurde bei einem sehr schwer löslichen Wirkstoff, nämlich dem Glibenclamid, eine Zubereitungsform beschrieben, bei der die Wirkstoffteilchen in ultrafeiner Form mit einer Teilchenoberfläche von 3-10 m²/g vorliegen und, eingebettet in den 2-20-fachen Überschuß an einem pharmakologisch verträglichen Netzmittel, bezogen auf den Wirkstoff, appliziert werden. Vorzugsweise wird als ein derartiges Netzmittel das nichtionische Polyoxiethylenstearat verwendet (vgl. DE-PS 23 48 334).

Die Herstellung von so fein zerteilten Wirkstoffen ist jedoch aufwendig, denn hierbei ist der Einsatz spezieller Mahlwerkzeuge notwendig bzw. muß der Wirkstoff in einer eigenen Verfahrensstufe aus einem Lösungsmittel in besonders feiner Form ausgefällt und dann getrocknet werden. Auch zeigen die hierbei zu verwendenden Netzmittel, die in einem hohen Überschuß verwendet werden sollen, oft unangenehme Nebenwirkungen, u. a. Hyperlipidämie, Erythrocytenaggregation, Hämolyse und Allergien.

Bei dem relativ schwer löslichen Wirkstoff 5-Aminosalicylsäure hat man gemäß DE-OS 31 51 196 dadurch gut lösliche Arzneimittelzubereitungen erhalten, daß man diese 5-Aminosalicylsäure zusammen mit basischen Hilfsstoffen, die in 1 %iger wäßriger Lösung pH-Werte von 8-12 ergaben, vermischt und diese Mischung zu Tabletten, Dragees, Kapseln oder Suppositorien verarbeitet. Beim Auflösen im Magen- und Darmtrakt bildet sich dann das leicht lösliche Salz der Aminosalicylsäure, wodurch eine schnelle Resorption erreicht werden kann.

Bekannt ist aus der DE-OS 23 55 743 auch, das sehr schwer lösliche Glibenclamid in Schmelzen von wasserlöslichen Trägerstoffen, vorzugsweise von Polyglykolen mit Molgewicht von 2000 bis 20000, gegebenenfalls in Gegenwart von oberflächenaktiven Stoffen und/oder wasserlöslichen Carrier-Stoffen sowie alkalischen Substanzen, aufzulösen und dann nach dem Abkühlen der Schmelze die Masse zu Granulaten zu verformen und in Arzneimitteldosierungen überzuführen.

Es wurde nun überraschenderweise festgestellt, daß man das Glibenclamid, auch wenn es nicht in sehr feinzerteilter Form vorliegt, sondern in einer üblichen Korngröße, die einer spezifischen Oberfläche von 0,8-1,4 m²/g entspricht, dann sehr gut und schnell resorbiert wird, wenn dieser Wirkstoff zusammen mit der basischen Verbindung Tris-(hydroximethyl)-aminomethan (Trometamol) und einem Polyethylenglykol mit Molekular gewicht von 6 000-35 000 in inniger mechanischer Mischung als Granulat oder Tablette verabreicht wird. Hierbei soll ein Mengenverhältnis von 1 Teil Wirkstoff : 1-20 Teilen Trometamol : 5-20 Teilen Polyethylenglykol eingehalten werden. Besonders gute Resorptionsgeschwindigkeiten wurden erreicht, wenn gleiche Mengen an Trometamol und Polyethylenglykol vorhanden sind, wobei eine optimale Wirkung bei einem Komponentenverhältnis von 1 : 10 : 10 festgestellt werden konnte.

Das Vermischen und Granulieren der drei Komponenten kann vorzugsweise unter Verwendung eines hochtourigen Mischers, z. B. eines sogenannten Fluidmischers erfolgen. Bei dieser intensiven Art der Vermischung kann sich das Gemisch erwärmen, weshalb durch Kühlung der Vorrichtung diese Erwärmung kontrolliert werden soll, damit die Temperatur der Mischung möglichst nicht über die Erweichungstemperatur des Polyethylenglykols ansteigt. Hierbei erhält man die Granulate, die als solche oder nach Tablettierung appliziert werden können.

Die Kombination von Wirkstoff und Polyethylenglykol allein führt nicht zu der gewünschten Verbesserung der Auflösung und Resorptionsgeschwindigkeit. Auch im Falle der Verwendung einer Applikationsform, die durch Bereiten einer klaren Schmelze des Wirkstoffs in erhitztem Polyethylenglykol bei Temperaturen von ca. 130 °C unter Auflösen des Wirkstoffs in dem Polyethylenglykol und anschließendem Abkühlen unter Auskristallisieren des Wirkstoffs und Wiederverfestigung des Polyethylenglykols hergestellt wurde, erhält man keine brauchbare schnell resorbierbare Zubereitungsform, weil, abgesehen von der Gefahr der thermischen Zersetzung des Wirkstoffs beim Abkühlen und bei der Lagerung, der Wirkstoff in unkontrollierter Form zu relativ großen Kristallen bzw. Teilchen auskristallisiert, so daß eine Standardisierung der Auflösungsgeschwindigkeit derartiger Präparate nicht möglich ist.

Durch die Granulierung unter Verwendung eines hochtourigen Mischers (Henschel-Fluidmischer) werden die erfindungsgemäß verwendeten Komponenten besonders intensiv vermischt, wobei die Temperaturerhöhung auf Temperaturen unter oder nur wenig über dem Erweichungspunkt des Polyethylenglykols zu halten ist. Hierdurch werden die Teilchen der Mischung agglomeriert und granuliert. Diese Granuliermethode ist an sich seit langem u. a. aus den Offenlegungsschriften 15 42 058 und 24 26 812 bzw. 24 26 811 bekannt. Hierbei wurden u. a. sogenannte Füllgranulate hergestellt, die dann mit einem Wirkstoffgranulat vermischt tablettiert werden sollen, um Retard-Tabletten zu erhalten. Mit dieser bei der erfindungsgemäßen Kombination angewendeten Granulierungsmethode wird nun erreicht, daß die Komponenten der Zubereitung, nämlich wasserunlöslicher Wirkstoff, Trometamol und Polyethylenglykol intensiv miteinander vermischt werden, um dann bei oraler Applikation des Granulats oder der daraus hergestellten Tabletten eine sehr schnelle Resorption des wasserunlöslichen Wirkstoffs sicherzustellen.

Bei dieser Verarbeitung wird die übliche Teilchengröße des Wirkstoffs nicht wesentlich verändert, dennoch erfolgt dessen rasche Auflösung im Magen- und Darmtrakt aufgrund der intensiven Vermischung mit dem Trometamol und dem Polyethylenglykol des beanspruchten Molekulargewichts. Es war nicht vorhersehbar, daß das Polyethylenglykol, welches kein Netzmittel darstellt, in Kombination mit der basischen Verbindung Trometamol eine starke Beschleunigung der Auflösung und Resorption der sehr schwer löslichen Wirkstoffe bewirkt, denn ohne Trometamol tritt dieser Effekt nicht ein. Aber auch hinsichtlich dieser basischen Verbindung ist ein Effekt vorhanden, der mit anderen basischen Verbindungen nicht erreicht werden kann. So bewirkt eine Zumischung von Natriumbicarbonat zu der Kombination aus schwer löslichem Wirkstoff und Polyethylenglykol des angegebenen Molekulargewichts nicht die gewünschte Löslichkeitsverbesserung.

Im Gegensatz zu den Ergebnissen, bei der aus der DE-PS 23 48 334 bekannten Kombination aus mikrofeinem Glibenclamidpulver und Netzmittel hat bei der erfindungsgemäßen Kombination die Feinheit der Teilchen des Wirkstoffs keinen so entscheidenden Einfluß auf die Löslichkeit und damit auf die Schnelligkeit der Resorption. Die mittlere Teilchengröße des schwer löslichen Wirkstoffs soll jedoch innerhalb des beanspruchten Bereichs liegen, da sich dann die Verbesserung und Erleichterung der Auflösung im Magen- und Darmtrakt am deutlichsten auswirkt. Die Verwendung eines stärker zerkleinerten, feineren Wirkstoffpulvers führt bei der erfindungsgemäßen Kombination zu keiner Steigerung der Löslichkeit.

Das neue erfindungsgemäße Prinzip der Löslichkeitsverbesserung von oral applizierbaren schwer löslichen Arzneimittelwirkstoffen durch Kombination mit Trometamol und polyethylenglykol ist bei den Verschiedensten pharmazeutischen Wirkstoffen anwendbar, die rasch resorbiert werden sollen, jedoch bei der oralen Applikation nur eine geringe Auflösungsgeschwindigkeit im Magen-Darm-Trakt zeigen. Außer bei Glibenclamid, bei dem der gewünschte Effekt besonders deutlich ist, bewährt sich die erfindungsgemäße Kombination auch bei anderen schwer löslichen Arzneimittelsubstanzen. Beispiele hierfür sind : Griseofulvin, Digoxin (Digitoxin), Diethylstilboestrol, Prednisolonacetat, Spironolacton, Indomethacin, Sulfathiazol, Hydrocortison, Kavain, Hexobarbital.

Durch die Verbesserung der Resorptionsgeschwindigkeit kann in vielen Fällen die gewünschte therapeutische Wirkung mit einer geringeren Dosis dieser Mittel erreicht werden als dies bisher der Fall war bzw. kann sie erreicht werden, ohne Gefahr von unerwünschten Nebenwirkungen, wie sie durch hohe Mengen an Netzmitteln oder dergl. auftreten können.

Im Folgenden soll die Erfindung anhand von Beispielen unter Verwendung des sehr schwer löslichen Wirkstoffs Glibenclamid näher erläutert werden.

Beispiel 1

In einem Henschel-Fluidmischer FM 200 wurden 1 891 g Glibenclamid mit einer Teilchengröße entsprechend 1 m²/g, 16 204 g Trometamol und 16 204 g Polyethylenglykol (Molgewicht 6 000) und 2 701 g Titandioxid als Weißpigment bei einer Tourenzahl von 500-800 Umdrehungen/Min. vermischt und granuliert, wobei durch Kühlung die Temperatur der Mischung auf maximal 50 °C gehalten wurde.

Das erhaltene Wirkstoffgranulat (37 000 g) wurde dann mit den üblichen Tablettierungsmitteln (Maisstärke, Cellulosepulver, Talkum, Polyvinylpyrrolidon, Siliciumdioxid — Aerosil A 200 — und Magnesiumstearat versetzt und das Gemisch zu 170 mg schweren Tabletten, enthaltend 3,5 mg Glibenclamid, verpreßt.

Beispiel 2

Wie im Beispiel 1 beschrieben wurde im Henschel-Fluidmischer 946 g Glibenclamid (1 m²/g), 16 677 g Trometamol und 16 677 g Polyethylenglykol (Mol-Gew. 6 000) vermischt und granuliert. Es wurden 34 300 g Granulat erhalten, das dann zusammen mit den Tablettierhilfsmitteln zu 170 mg schweren Tabletten, enthaltend 1,75 mg Glibenclamid, verpreßt wurde.

**Patentansprüche**

1. Orale, schnell resorbierbare Zubereitung schwer löslicher Arzneimittelwirkstoffe, gekennzeichnet durch eine innige mechanische Mischung von Glibenclamid einer Teilchenoberfläche von 0,8-1,4 m²/g mit Tris-(hydroxymethyl)-aminomethan (Trometamol) und einem festen Polyethylenglykol mit mittlerem Molekulargewicht von 6 000-35 000.

2. Zubereitung nach Anspruch 1, dadurch gekennzeichnet, daß das Mischungsverhältnis von Glibenclamid zu Trometamol zu Polyethylenglykol 1 : 1-20 : 5-20 beträgt.

3. Zubereitung nach Anspruch 1-3, dadurch gekennzeichnet, daß die Mischung in Form eines Granulats oder daraus hergestellter Tablette vorliegt.

4. Verfahren zur herstellung einer oralen Zubereitung nach Anspruch 1-3, dadurch gekennzeichnet, daß man das Glibenclamid, das Trometamol und das Polyethylenglykol in einem Fluidmischer bei Temperaturen unter dem Erweichungspunkt des Polyethylenglykols intensiv vermischt und granuliert.

5. Verfahren nach Anspruch 4, dadurch gekennzeichnet, daß man das erhaltene Granulat in an sich bekannter Weise zu Tabletten verpreßt.

## Claims

1. Rapidly absorbable oral preparation of pharmaceutically active agents that are difficult to dissolve, characterized by an intimate mechanical mixture of Glibenclamid having a particle surface of from 0.8 to 1.4 $m^2/g$ and tris(hydroxymethyl)amino methane (Trometamol) and a solid polyethylene glycol having an average molecular weight of from 6 000 to 35 000.

2. The preparation as defined in claim 1, characterized in that the mixing ratio of Glibenclamid to Trometamol to polyethylene glycol is 1 : 1-20 : 5-20.

3. The preparation as defined in claims 1 to 2, characterized in that the mixture is present in the form of a granulate or a tablet prepared from such granulate.

4. A process for producing an oral preparation as defined in claims 1 to 3, characterized in that the Glibenclamid, the Trometamol and the polyethylene glycol are intimitely mixed and granulated in a fluid mixer at temperatures below the softening point of the polyethylene glycol.

5. The process as defined in claim 4, characterized in that the obtained granulate is pressed to tablets in a manner known per se.

## Revendications

1. Composition orale, à résorption rapide, de substances actives médicamenteuses peu solubles, caractérisée par un mélange mécanique intime de Glibenclamid à une surface de particule de 0,5 à 1,4 $m^2/g$ avec du tris-(hydroxyméthyl)-aminométhane (Trometamol) et un polyéthylèneglycol solide de poids moléculaire moyen 6 000 à 35 000.

2. Composition selon la revendication 1, caractérisée en ce que les proportions relatives de mélange Glibenclamid/Trometamol/polyéthylèneglycol sont de 1 : 1 à 20 : 5 à 20.

3. Composition selon la revendication 1 ou 2, caractérisée en ce que le mélange est à l'état de granulés ou de comprimés préparés à partir des granulés.

4. Procédé de préparation d'une composition orale selon les revendications 1 à 3, caractérisé en ce que l'on soumet le Glibenclamid, le Trometamol et le polyéthylèneglycol à mélange intensif et mise en granulés dans un mélangeur à fluidisation à des températures inférieures au point de ramollissement du polyéthylèneglycol.

5. Procédé selon la revendication 4, caractérisé en ce que l'on met les granulés obtenus à la presse, de manière connue en soi, à l'état de comprimés.